# EUROPÄISCHE PATENTSCHRIFT

(11) **EP 0 346 708 B1**
(45) Veröffentlichungstag und Bekanntmachung des Hinweises auf die Patenterteilung: **10.02.1993**
(21) Anmeldenummer: 89110093.5
(22) Anmeldetag: 03.06.1989
(51) Int. Cl.: A61K 6/08

(54) **Füllstoffe**
Fillers
Charges

(30) Priorität: 16.06.1988 DE 3820497
(43) Veröffentlichungstag der Anmeldung: 20.12.1989
(73) Patentinhaber: BAYER AG, 51368 Leverkusen (DE)
(72) Erfinder: Podszun, Wolfgang, Dr., D-5000 Köln 80 (DE); Reiners, Jürgen, Dr., D-5090 Leverkusen 1 (DE)

(56) Entgegenhaltungen:
- EP-A- 0 011 761
- EP-A- 0 032 124
- EP-A- 0 084 769
- DE-A- 1 907 666
- US-A- 4 396 377

## Beschreibung

Die Erfindung betrifft polymere vernetzte (Meth)-Acrylate für Anwendungen im Dentalbereich, ihre Herstellung und ihre Verwendung in Dentalwerkstoffen.

Füllstoffe für Anwendungen im Dentalbereich können beispielsweise bei der Herstellung von künstlichen Zähnen, Kronen, Brücken, Inlays, Onlays, Zahnfüllungen und Zahnlacken eingesetzt werden. Es werden hierbei sowohl anorganische Füllstoffe, wie feingemahlener Quarz, als auch organische, polymere Füllstoffe verwendet. Im Vergleich zu anorganischen Füllstoffen führen organische Füllstoffe zu homogeneren Dentalwerkstoffen.

Als organische Füllstoffe seien beispielsweise vernetzte Perlpolymerisate mit einer Teilchengröße von 10 bis 200 µm genannt (DE-A 28 49 280), die jedoch nur wenig von den Monomeren der polymerisierbaren Dentalwerkstoffe gelöst und angequollen werden; damit ist noch keine völlige Homogenität entsprechender Dentalwerkstoffe gegeben.

Durch das Anquellen vernetzter organischer Polymere kann man sogenannte IPN-Polymere (interpenetrating network) herstellen (J. Polper Science 12, 141 (1977) und J. Polymer Science 16, 583 (1978)), die sich durch hohe Festigkeit und Lösemittelbeständigkeit auszeichnen.

Füllstoffe für Anwendungen im Dentalbereich aus Perlpolymerisaten mit einem Quellungsgrad von 10 bis 50 Gew.-% werden in der WO 82/02556 beschrieben Diese Perlpolymerisate haben jedoch nur geringe Vernetzergehalte und erreichen nicht die hohe mechanische Festigkeit höher vernetzter Polymerer.

Es wurden Füllstoffe für Anwendungen im Dentalbereich, enthaltend polymere vernetzte (Meth)-Acrylate mit einer Teilchengröße im Bereich von 0,01 bis 10 µm gefunden, die dadurch gekennzeichnet sind, daß sie einen Quellungsgrad von 100 bis 2000 Gew.-% und einen Vernetzungsgrad von 50 bis 100 Gew.-%, jeweils bezogen auf das Polymer, aufweisen

(Meth)acrylate im Rahmen der vorliegenden Erfindung sind Ester der Acrylsäure und/oder Methacrylsäure. Bevorzugt sind Ester der Methacrylsäure.

Die erfindungsgemäßen Poly(meth)acrylate weisen einen Vernetzungsgrad von 50 bis 100 Gew.-%, bevorzugt von 85 bis 100 Gew-%, auf. Als Vernetzungsgrad wird der Anteil an Methacrylsäureestern, die Vernetzungen ausbilden können, definiert.

Monomere (Meth)-Acrylate, die Vernetzungen ausbilden, sind (Meth)-Acrylate mit 2 oder mehr, bevorzugt 2 bis 4, polymerisierbaren Doppelbindungen im Molekül.

Als monomere (Meth)-Acrylate, die Vernetzungen ausbilden, seien beispielsweise genannt:
Ethylenglykoldimethacrylat, Diethylenglykoldimethacrylat, Triethylenglykoldimethacrylat, Glycerindimethacrylat, Glycerintrimethacrylat, Trimethylolpropantrimethacrylat, Pentaerythritdimethacrylat, Pentaerythrittrimethacrylat, Pentaerythrittetramethacrylat, Derivate des Bisphenol A, wie Bisphenol-A-dimethacrylat und Bisphenol-A-diglykoldimethacrylat, Urethanmethacrylate, die durch Umsetzung von Diisocyanaten und Hydroxyalkylmethacrylaten hergestellt werden können, wie 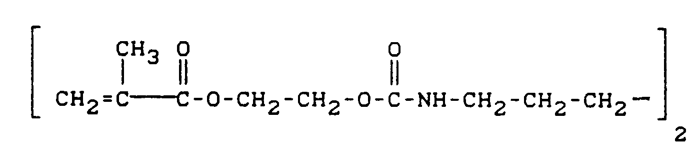
Umsetzungsprodukte aus Polyolen, Diisocyanaten und Hydroxyalkylmethacrylaten (DE-A 37 03 080, DE-A 37 03 130 und DE-A 37 03 120).

Bevorzugt werden monomere (Meth)-Acrylate, die Vernetzungen ausbilden, wie:
Ethylenglycoldimethacrylat, Diethylenglycoldimethacrylat, Triethylenglycoldimethacrylat und Glycerindimethacrylat.

Als monomere (Meth)-Acrylate, die keine Vernetzungen ausbilden, seien beispielsweise genannt:
C₁-C₁₂-, bevorzugt C₁-C₄-Alkylmethacrylate, wie Methylmethacrylat, Ethylmethacrylat, n-Propylmethacrylat, iso-Propylmethacrylat, n-Butylmethacrylat, t-Butylmethacrylat,
Hydroxyalkyl(C₁-C₄)methacrylate, wie 2-Hydroxyethylmethacrylat, 2-Hydroxypropylmethacrylat, Diethylenglykolmonomethacrylat, Triethylenglykolmonomethacrylat,
Alkoxy(C₁-C₄)alkylmethacrylate, wie 2-Methoxyethylmethacrylat, 3-Methoxybutylmethacrylat und Ethyltriglykolmethacrylat.

Bevorzugte monomere (Meth)-Acrylate, die keine Vernetzungen ausbilden, sind beispielsweise:
Methylmethacrylat, Ethylmethacrylat, 2-Hydroxyethylmethacrylat.

Die monomeren (Meth)-Acrylate sind an sich bekannt und können beispielsweise durch Umsetzung von (Meth)-Acrylsäurechlorid mit den entsprechenden Alkoholen hergestellt werden.

Es ist selbstverständlich möglich, daß die erfindungsgemäßen (Meth)-Acrylate mit weiteren Monomeren Copolymere bilden. Beispielsweise seien hier Copolymere mit Styrol, α-Methylstyrol, Acrylnitril und Vinylacetat genannt. In diesen Fällen beträgt der Anteil des Comonomers 0 bis 40, bevorzugt 0 bis 20 Gew.-%, bezogen auf das Polymer.

Unter dem Quellungsgrad versteht man das Aufnahmevermögen der erfindungsgemäßen Poly(meth)acrylate an Flüssigkeit. Der Quellungsgrad wird gemessen durch das Aufnahmevermögen an Tetrahydrofuran bei 20°C. Die erfindungsgemäßen Poly(meth)acrylate weisen eine Aufnahme an Tetrahydrofuran von 50 bis 2000 Gew.-%, bevorzugt von 100 bis 1000 Gew.-%, bezogen auf das Polymer, auf.

Die erfindungsgemäßen Poly(meth)acrylate weisen im allgemeinen einen mittleren Teilchendurchmesser von 0,01 bis 10 µm, bevorzugt von 0,05 bis 5 µm, auf.

Bevorzugt weisen die erfindungsgemäßen Poly(meth)acrylate einen Gelgehalt von 90 bis 100 Gew.-%, bevorzugt von 95 bis 100 Gew.-%, bezogen auf das Polymer, auf. Im Rahmen der vorliegenden Erfindung versteht man unter dem Gelgehalt definitionsgemäß den Anteil des Polymers, der mit THF als Lösungsmittel bei 20°C nicht löslich ist. Der Gelgehalt ist eine Kenngröße für die tatsächlich eingetretene Vernetzung.

Die erfindungsgemäßen Poly(meth)acrylate weisen bevorzugt eine aktive Oberfläche von 20 bis 600 m²/g, bevorzugt von 50 bis 300 m²/g (gemessen nach der BET-Methode) auf.

Es wurde auch ein Verfahren zur Herstellung der erfindungsgemäßen Poly(meth)acrylate für Anwendungen im Dentalbereich gefunden, das dadurch gekennzeichnet ist, daß man monomere (Meth)-Acrylate, die Vernetzungen ausbilden, und gegebenenfalls (Meth)-Acrylate, die keine Vernetzungen ausbilden, und gegebenenfalls weitere Comonomere in Gegenwart eines organischen Lösungsmittels mit einem Löslichkeitsparameter von 8 bis 15 [cal⁰ʼ⁵cm⁻¹ʼ⁵] polymerisiert, wobei der Monomeranteil der (Meth)-Acrylate, die Vernetzungen ausbilden, 50 bis 100 Gew.-% beträgt.

Organische Lösungsmittel können durch den sogenannten Löslichkeitsparameter definiert werden (H.G. Elias, Makromoleküle, S. 192-196 (1981)). Für das erfindungsgemäße Verfahren verwendet man Lösungsmittel mit einem Parameter von 8 bis 15 [cal⁰ʼ⁵cm⁻¹ʼ⁵], bevorzugt von 8,5 bis 12 [cal⁰ʼ⁵cm⁻¹ʼ⁵].

Beispielsweise seien die folgenden Lösungsmittel genannt:
Amylacetat, Tetrachlorethan, Toluol, Ethylacetat, Tetrahydrofuran, Benzol, Trichlormethan, Dichlormethan, Methylchlorid, Aceton, Butanon-2 und tert.-Butanol.

Die Menge des Lösungsmittels im Verhältnis zu den monomeren (Meth)-Acrylaten, die Vernetzungen ausbilden können, liegt im Bereich von 1:1 bis 1:100, vorzugsweise 1:2 bis 1:20.

Die Durchführung des erfindungsgemäßen Polymerisationsverfahrens erfolgt im allgemeinen im Temperaturbereich von 50 bis 250°C, bevorzugt von 60 bis 150°C. Dabei kann die Polymerisation kontinuierlich oder diskontinuierlich durchgeführt werden.

Die Polymerisation wird im allgemeinen in Gegenwart von Initiatoren wie Sensibilisatoren oder Radikalbildnern durchgeführt.

Die Initiatoren werden im allgemeinen in einer Menge von 0,01 bis 3 Gew.-%, vorzugsweise 0,1 bis 1,5 Gew.-%, bezogen auf das Gesamtmonomere, eingesetzt.

Als Polymerisationsinitiatoren können beispielsweise Perverbindungen oder Radikale liefernde Azoverbindungen verwendet werden. Beispielsweise seien genannt:
Aliphatische Azodicarbonsäurederivate wie Azobisisobuttersäurenitril oder Azodicarbonsäureester, Peroxide wie Lauroylperoxid, Succinylperoxid, Dibenzoylperoxid, p-Chlorbenzoylperoxid, 2,4-Dichlorbenzoylperoxid, Peroxide wie Methylethylketonperoxid, Methylisobutylketonperoxid, Cyclohexanonperoxid, Acetylacetonperoxid, Alkylester von Persäuren wie tert.-Butylperpivalat, tert.-Butylperoctoat, tert.-Butylperbenzoat, tert.-Butylperisononalat, Mono-tert.-Butylpermaleinat, tert.-Butylperacetat, Percarbonate wie Dicyclohexyl- und Diisopropylpercarbonat, Dialkylperoxide wie Di-tert.-Butylperoxid, Dicumylperoxid, Hydroperoxide wie tert.-Butyl- oder Cumolhydroperoxide, Isophthal-monopersäure oder Acetylcyclohexansulfonylperoxid.

Bei der erfindungsgemäßen Polymerisation entsteht im allgemeinen eine Suspension des Füllstoffs. Die Isolierung des Füllstoffs kann beispielsweise durch Verdampfen des Lösungsmittels, beispielsweise in einem Sprühtrocknungsprozeß, erfolgen.

Die erfindungsgemäßen Füllstoffe werden als Komponenten für Dentalwerkstoffe verwendet. Sie sind gut benetzbar, leicht dispergierbar und lassen sich in einfacher Weise in Dentalmonomere einarbeiten. Als bevorzugte Dentalmonomere seien beispielsweise genannt:
Methylmethacrylat, Ethylendimethacrylat, Diethylenglycoldimethacrylat, Triethylglycoldimethacrylat, Bisphenol-A-diglycidyldimethacrylat und Mischungen der genannten Monomere.

Bevorzugte Dentalmassen enthalten 5 - 35 Gew.-Teile der erfindungsgemäßen Füllstoffe, 40 - 90 Gew.-Teile Dentalmonomere und 0,1 bis 10 Gew.-Teile übliche Zusätze wie Initiatoren, Pigmente und Stabilisatoren.

Zur Herstellung von Kunststoffzähnen ist beispielsweise eine Mischung aus 10 - 25 % erfindungsgemäßen Füllstoff, 50 - 60 % Bisphenol-A-diglycedyldimethacrylat, 25 - 30 % Triethylenglycoldimethacrylat und 0,5 % Dibenzoylperoxid besonders gut geeignet.

Anwendungsformen im Dentalbereich, z.B. künstliche Zähne, Kronen oder Zahnfüllungen, die den erfindungsgemäßen Füllstoff enthalten, zeichnen sich durch hohe mechanische Festigkeit, vor allem hohe Abrasionsresistenz, sowie hohe Transparenz aus.

### Beispiel 1

In einem 3 l-Glasreaktor, der mit Blattrührer, Rückflußkühler, Innenthermometer, Gaseinlaß und Gasauslaßrohr ausgerüstet ist, werden 1800 g Butanon-2, 200 g Ethylenglykoldimethacrylat und 2 g Dibenzoylperoxid eingewogen. Unter Rühren mit 300 UpM und unter Stickstoffspülung wird 2 Stunden am Rückfluß erhitzt. Es entsteht eine gut rührbare Suspension. Aus dieser lassen sich durch Sprühtrocknung 190 g feines Pulver gewinnen. Die mittlere Teilchengröße (gemessen mit Laserkorrelationsspektroskopie) beträgt 700 nm, der Gelgehalt 98,4 %, der Quellungsgrad (gemessen in THF) 310 % und die BET-Oberfläche 155 m²/g.

### Beispiel 2

Beispiel 1 wird wiederholt, wobei anstelle von Ethylenglykoldimethacrylat, Triethylenglykoldimethacrylat eingesetzt wird. Man erhält 196 g feines Pulver mit einer Teilchengröße von 500 nm, einem Gelgehalt von 95,8 %, einem Quellungsgrad von 690 % und einer BET-Oberfläche von 120 m²/g.

### Beispiel 3

Beispiel 1 wird wiederholt, wobei anstelle von Ethylenglykoldimethacrylat, Trimethylolpropantrimethacrylat eingesetzt wird. Man erhält 192 g Pulver mit einer Teilchengröße von 900 nm, einem Gelgehalt von 97,1 % und einem Quellungsgrad von 120 %.

### Beispiel 4

Nach der in Beispiel 1 angegebenen Arbeitsweise wurden 500 g Glycerindimethacrylat und 5 g Dibenzoylperoxid in 2000 g Butanon-2 umgesetzt. Man erhält 475 g Pulver mit einer Teilchengröße von 350 nm, einem Gelgehalt von 97,3 %, einem Quellungsgrad von 280 % und einer BET-Oberfläche von 128 m²/g.

### Beispiel 5

### Polymerisierbare Masse

104 g Polymerisat aus Beispiel 1, 248 g Bis-GMA, 152 g Triethylenglykoldimethacrylat und 2,1 g Dibenzoylperoxid werden in einem Laborkneter in 30 Min. verknetet. Die erhaltene Masse wird 5 Stunden bei 35°C gelagert. Man erhält eine transparente, nicht klebende, teigartige Paste.

### Beispiel 6

### Polymerisierbare Masse

36 g Polymerisat aus Beispiel 2, 120 g des Umsetzungsproduktes aus 2,2,4-Trimethylhexamethylendiisocyanat und 2 Mol 2-Hydroxyethylmethacrylat [1,6-Bis-(Methacrylolyoxyethoxycarbonylamino)-2,2,4-trimethylhexan] und 0,64 g Dibenzoylperoxid werden wie im Beispiel 3 beschrieben zu einer Masse verknetet.

### Beispiel 7

20 g Polymerisat aus Beispiel 2, 60 g Bis-GMA, 30 g Triethylenglykoldimethacrylat und 10 g 4-Methoxybutylmethacrylat und 0,5 g Dibenzoylperoxid werden wie in Beispiel 3 beschrieben zu einer Masse verknetet.

## Patentansprüche (Patentansprüche für folgende(n) Vertragsstaat(en): AT, BE, CH, DE, FR, GB, GR, IT, LI, LU, NL, SE)

1. Polymere vernetzte (Meth)-Acrylate mit einer Teilchengröße im Bereich von 0,01 bis 10 µm, dadurch gekennzeichnet, daß sie einen Quellungsgrad von 100 bis 2000 Gew.-% und einen Vernetzungsgrad von 50 bis 100 Gew.-%, jeweils bezogen auf das Polymer, aufweisen.

2. Polymere nach Anspruch 1, dadurch gekennzeichnet, daß sie einen Gelgehalt von 90 bis 100 Gew.-% aufweisen.

3. Polymere nach den Ansprüchen 1 und 2, dadurch gekennzeichnet, daß sie eine aktive Oberfläche im Bereich von 20 bis 600 m²/g aufweisen.

4. Verfahren zur Herstellung von polymeren vernetzten (Meth)-Acrylaten gemäß Ansprüchen 1 - 3, dadurch gekennzeichnet, daß man monomere (Meth)-Acrylate, die Vernetzungen ausbilden, und gegebenenfalls (Meth)-Acrylate, die keine Vernetzungen ausbilden, und gegebenenfalls weitere Comonomere in Gegenwart von organischen Lösungsmitteln mit einem Löslichkeitsparameter von 8 bis 15 [cal^{0,5}cm^{-1,5}] polymerisiert, wobei der Monomeranteil der (Meth)-Acrylate, die Vernetzungen ausbilden, 50 bis 100 Gew.-% beträgt.

5. Verfahren nach Anspruch 4, dadurch gekennzeichnet, daß die Menge des Lösungsmittels im Verhältnis zu den monomeren (Meth)-Acrylaten, die Vernetzungen ausbilden können, im Bereich von 1:1 bis 1:100 liegt.

6. Verwendung von polymeren vernetzten (Meth)-Acrylaten nach den Ansprüchen 1 bis 3 zur Herstellung von Dentalmaterialien.

7. Verwendung von polymeren vernetzten (Meth)-Acrylaten nach den Ansprüchen 1 bis 3 zur Herstellung von Füllstoffen.

8. Verwendung von polymeren vernetzten (Meth)-Acrylaten nach den Ansprüchen 1 bis 3 zur Herstellung von künstlichen Zähnen.

## Patentansprüche (Patentansprüche für folgende(n) Vertragsstaat(en): ES)

1. Verfahren zur Herstellung von polymeren vernetzten (Meth)-Acrylaten mit einer Teilchengröße im Bereich von 0,01 bis 10 µm, einem Quellungsgrad von 100 bis 2000 Gew.-% und einem Vernetzungsgrad von 50 bis 100 Gew.-%, jeweils bezogen auf das Polymer, dadurch gekennzeichnet, daß man monomere (Meth)-Acrylate, die Vernetzungen ausbilden, und gegebenenfalls (Meth)-Acrylate, die keine Vernetzungen ausbilden, und gegebenenfalls weitere Comonomere in Gegenwart von organischen Lösungsmitteln mit einem Löslichkeitsparameter von 8 bis 15 [cal^{0,5}cm ^{-1,5}] polymerisiert, wobei der Monomeranteil der (Meth-)Acrylate, die Vernetzungen ausbilden, 50 bis 100 Gew.-% beträgt.

2. Verfahren nach Anspruch 2, dadurch gekennzeichnet, daß die Menge des Lösungsmittels im Verhältnis zu den monomeren (Meth)-Acrylaten, die Vernetzungen ausbilden können, im Bereich von 1:1 bis 1:100 liegt.

## Claims (Claims for the following Contracting State(s): AT, BE, CH, DE, FR, GB, GR, IT, LI, LU, NL, SE)

1. Polymeric cross-linked (meth)-acrylates having a particle size in the range from 0.01 to 10 µm, characterised in that they have a degree of swelling of 100 to 2,000 % by weight and a degree of cross-linking of 50 to 100 % by weight, in each case relative to the polymer.

2. Polymers according to Claim 1, characterised in that they have a gel content of 90 to 100 % by weight.

3. Polymers according to Claims 1 and 2, characterised in that they have an active surface area in the range from 20 to 600 m²/g.

4. Process for the preparation of polymeric cross-linked (meth)-acrylates according to Claims 1 to 3, characterised in that monomeric (meth)-acrylates which form cross-linkages and, if appropriate, (meth)-acrylates which form no cross-linkages and, if appropriate, further comonomers are polymerized in the presence of organic solvents having a solubility parameter of 8 to 15 [cal^{0,5} cm^{-1,5}], the monomer proportion of the (meth)-acrylates which form cross-linkages being 50 to 100 % by weight.

5. Process according to Claim 4, characterised in that the amount of solvent in proportion to the monomeric (meth)-acrylates which can form cross-linkages is in the range from 1:1 to 1:100.

6. Use of polymeric cross-linked (meth)-acrylates according to Claim 1 to 3 for the production of dental materials.

7. Use of polymeric cross-linked (meth)-acrylates according to Claims 1 to 3 for the production of fillers.

8. Use of polymeric cross-linked (meth)-acrylates according to Claims 1 to 3 for the production of false teeth.

## Claims (Claims for the following Contracting State(s): ES)

1. Process for the preparation of polymeric cross-linked (meth)-acrylates having a particle size in the range from 0.01 to 10 µm, a degree of swelling of 100 to 2,000 % and a degree of cross-linking of 50 to 100 % by weight, in each case relative to the polymer, characterised in that monomeric (meth)-acrylates which form cross-linkages and, if appropriate, (meth)-acrylates which form no cross-linkages and, if appropriate, further comonomers are polymerized in the presence of organic solvents having a solubility parameter of 8 to 15 (cal^{0,5} cm^{-1,5}], the monomer proportion of the (meth)-acrylates which form cross-linkages being 50 to 100 % by weight.

2. Process according to Claim 1, characterised in that the amount of solvent in proportion to the monomeric (meth)-acrylates which can form cross-linkages is in the range from 1:1 to 1:100.

## Revendications (Revendications pour l'(les) Etat(s) contractant(s) suivant(s): AT, BE, CH, DE, FR, GB, GR, IT, LI, LU, NL, SE)

1. (Méth)-acrylates réticulés polymériques ayant un diamètre de particules dans la plage de 0,01 à 10 µm, caractérisés en ce qu'ils présentent un degré de gonflement de 100 à 2000 % en poids et un degré de réticulation de 50 à 100 % en poids, dans chaque cas par rapport au polymère.

2. Polymères suivant la revendication 1, caractérisés en ce qu'ils présentent une teneur en gel de 90 à 100 % en poids.

3. Polymères suivant les revendications 1 et 2, caractérisés en ce qu'ils présentent une surface active dans la plage de 20 à 600 m²/g.

4. Procédé de production de (méth)-acrylates réticulés polymériques suivant les revendications 1 à 3, caractérisé en ce qu'on polymérise des (méth)-acrylates monomères qui créent des réticulations et le cas échéant des (méth)-acrylates qui ne créent pas de réticulation, avec éventuellement d'autres comonomères en présence de solvants organiques ayant un paramètre de solubilité de 8 à 15[cal^{0,5}cm^{-1,5}], la proportion de (méth)-acrylates monomères qui créent des réticulations s'élevant à 50-100 % en poids.

5. Procédé suivant la revendication 4, caractérisé en ce que la quantité de solvant par rapport aux (méth)-acrylates monomères qui peuvent créer des réticulations se situe dans la plage de 1:1 à 1:100.

6. Utilisation de (méth)-acrylates réticulés polymériques suivant les revendications 1 à 3 pour la production de matériaux dentaires.

7. Utilisation de (méth)-acrylates réticulés polymériques suivant les revendications 1 à 3 pour la production de charges.

8. Utilisation de (méth)-acrylates réticulés polymériques selon les revendications 1 à 3 pour la production de dents artificielles.

## Revendications (Revendications pour l'(les) Etat(s) contractant(s) suivant(s): ES)

1. Procédé de production de (méth)-acrylates réticulés polymériques ayant un diamètre de particules dans la plage de 0,01 à 10 µm, un degré de gonflement de 100 à 2000 % en poids et un degré de réticulation de 50 à 100 % en poids, dans chaque cas par rapport au polymère, caractérisé en ce qu'on polymérise des (méth)-acrylates monomères qui créent des réticulations, et le cas échéant des (méth)-acrylates qui ne créent pas de réticulation, avec éventuellement d'autres comonomères en présence de solvants organiques ayant un paramètre de solubilité de 8 à 15[cal^{0,5}cm^{-1,5}], la proportion de (méth)-acrylates monomères qui créent des réticulations s'élevant à 50-100 % en poids.

2. Procédé suivant la revendication 1, caractérisé en ce que la quantité du solvant par rapport aux (méth)-acrylates monomères qui peuvent créer des réticulations se situe dans la plage de 1:1 à 1:100.
